# EUROPÄISCHE PATENTSCHRIFT

(11) **EP 1 367 129 B1**
(45) Veröffentlichungstag und Bekanntmachung des Hinweises auf die Patenterteilung: **01.07.2009**
(21) Anmeldenummer: 03010226.3
(22) Anmeldetag: 07.05.2003
(51) Int. Cl.: C12P 13/04, C12P 41/00

(54) **Verfahren zur enzymatischen Herstellung enantiomerenangereicherter Beta-Aminosäuren**
Method for the enzymatic preparation of enantiomerically-enriched beta-amino acids
Procédé pour la préparation enzymatique de beta amino acides enrichis en énantiomères

(30) Priorität: 08.05.2002 DE 10220740
(43) Veröffentlichungstag der Anmeldung: 03.12.2003
(73) Patentinhaber: Evonik Degussa GmbH, 45128 Essen (DE)
(72) Erfinder: Gröger, Harald, Dr., 63450 Hanau (DE); Werner, Helge, 63486 Bruchköbel (DE)

(56) Entgegenhaltungen:
- US-A- 4 636 470
- FAULCONBRIDGE S J ET AL: "Preparation of enantiomerically enriched aromatic beta-amino acids via enzymatic resolution" TETRAHEDRON LETTERS, ELSEVIER SCIENCE PUBLISHERS, AMSTERDAM, NL, Bd. 41, Nr. 15, April 2000 (2000-04), Seiten 2679-2681, XP004194578 ISSN: 0040-4039

## Beschreibung

Die vorliegende Erfindung bezieht sich auf ein Verfahren zur Herstellung von enantiomerenangereicherten β-Aminosäuren.

Optisch aktive β-Aminocarbonsäuren treten in Naturstoffen wie Alkaloiden und Antibiotika auf, und deren Isolierung gewinnt zunehmend an Interesse, nicht zuletzt wegen deren steigender Bedeutung als essentielle Zwischenprodukte bei der Herstellung von Arzneimitteln (siehe u.a.: E. Juaristi, H. Lopez-Ruiz, *Curr. Med. Chem.* 1999, *6*, 983-1004). Sowohl die freie Form optisch aktiver β-Aminocarbonsäuren als auch deren Derivate zeigen interessante pharmakologische Effekte und können auch bei der Synthese modifizierter Peptide eingesetzt werden.

Als Herstellungsmethoden für β-Aminocarbonsäuren haben sich bislang die klassische Racematspaltung über diastereomere Salze (vorgeschlagene Route in: H. Boesch et al., Org. Proc. Res. Developm. 2001, 5, 23-27) und insbesondere die diastereoselektive Addition von Lithium-Phenylethylamid (A. F. Abdel-Magid, J. H. Cohen, C. A. Maryanoff, *Curr. Med*. *Chem*. 1999, *6*, 955-970) etabliert. Letztere Methode gilt als intensiv erforscht und wird trotz zahlreicher dabei auftretender Nachteile bevorzugt angewandt. Zum einen werden stöchiometrische Mengen eines chiralen Reagenzes benötigt, was im Vergleich zu katalytischen asymmetrischen Methoden einen großen Nachteil darstellt. Außerdem werden teure und zudem gefährliche Hilfsstoffe wie beispielsweise *n*-Butyllithium zur Aktivierung des stöchiometrischen Reagenz durch Deprotonierung benötigt. Für eine genügende Stereoselektivität ist zudem die Durchführung der Reaktion bei niedrigen Temperaturen von ca. -70°C wichtig, was einen hohen Anspruch an das Reaktormaterial, zusätzliche Kosten und einen hohen Energieverbrauch bedeutet.

Die Herstellung von optisch aktiven β-Aminocarbonsäuren auf biokatalytischem Wege spielt zwar gegenwärtig nur eine untergeordnete Rolle, ist aber insbesondere aufgrund der ökonomischen und ökologischen Vorteile biokatalytischer Reaktionen wünschenswert. Es entfällt der Einsatz stöchiometrischer Mengen eines chiralen Reagenzes und stattdessen werden geringe, katalytische Mengen von Enzymen eingesetzt, die natürliche und umweltfreundliche Katalysatoren darstellen. Diese im wässrigen Medium effizient eingesetzten Biokatalysatoren weisen neben ihren katalytischen Eigenschaften und ihrer hohen Wirksamkeit zudem, im Gegensatz zu einer Vielzahl von synthetischen metallhaltigen Katalysatoren, den Vorteil auf, dass auf die Verwendung metallhaltiger, insbesondere schwermetallhaltiger und somit toxischer Einsatzstoffe verzichtet werden kann.

Im Stand der Technik wurde z.B. bereits mehrfach über die enantioselektive *N*-Acylierung von β-Aminocarbonsäuren berichtet.

So beschreiben L.T. Kanerva et al. in Tetrahedron: Asymmetry, Vol. 7, No. 6, S. 1707-1716, 1996 die enantioselektive *N*-Acylierung von Ethylestern verschiedener alicylischer β-Aminocarbonsäuren mit 2,2,2-Trifluorethylester in organischen Lösungsmitteln und Lipase SP 526 aus *Candida antarctica* oder Lipase PS aus *Pseudomonas cepacia* als Biokatalysator.

V.M. Sánchez et al. untersuchten die biokatalytische Racematspaltung von (±)-Ethyl-3-aminobutyrat (Tetrahedron: Asymmetry, Vol. 8, No. 1, S. 37-40, 1997) mit Lipase aus *Candida antarctica* über die Herstellung des *N*-acetylierten β-Aminocarbonsäureesters.

In EP-A-8 890 649 ist ein Verfahren zur Herstellung von optisch aktiven Aminosäureestern aus racemischen Aminosäureestern durch enantioselektive Acylierung mit einem Carbonsäureester in Gegenwart einer Hydrolase, ausgewählt aus der Gruppe Amidase, Protease, Esterase und Lipase, und nachfolgende Isolierung des nicht umgesetzten Enantiomers des Aminosäureesters offenbart.

WO-A-98/50575 betrifft ein Verfahren zur Gewinnung einer chiralen β-Aminocarbonsäure oder ihrer entsprechenden Ester durch Inberührungbringen einer racemischen β-Aminocarbonsäure, eines Acyldonors und Penicilin G Acylase unter Bedingungen, um ein Enantiomer der racemischen β-Aminocarbonsäure stereoselektiv zu acylieren, wobei das andere Enantiomer im wesentlichen nicht umgesetzt wird, und man so eine chirale β-Aminocarbonsäure erhält.
Auch die umgekehrte Reaktionsfolge ist untersucht worden (V. A. Soloshonok, V. K. Svedas, V. P. Kukhar, A. G. Kirilenko, A. V. Rybakova, V. A. Solodenko, N. A. Fokina, O. V. Kogut, I. Y. Galaev, E. V. Kozlova, I. P. Shishkina, S. V. Galushko, Synlett 1993, 339-341; V. Soloshonok, A. G. Kirilenko, N. A. Fokina, I. P. Shishkina, S. V. Galushko, V. P. Kukhar, V. K. Svedas, E. V. Kozlova, Tetrahedron: Asymmetry 1994, 5, 1119-1126; V. Soloshonok, N. A. Fokina, A. V. Rybakova, I. P. Shishkina, S. V. Galushko, A. E. Sochorinsky, V. P. Kukhar, M. V. Savchenko, V. K. Svedas, Tetrahedron: Asymmetry 1995, 6, 1601-1610; G. Cardillo, A. Tolomelli, C. Tomasini, Eur. J. Org. Chem. 1999, 155-161). Nachteilig bei diesem Verfahren ist die schwierige Aufarbeitung des Produktgemisches nach der enantioselektiven Hydrolyse. Nach Abtrennung der freien β-Aminocarbonsäure erhält man ein Gemisch aus Phenylessigsäure und *N*-Phenylacetyl-β-Aminocarbonsäure, das schwierig aufzutrennen ist.

Zur Gewinnung von enantiomerenangereicherten Carbonsäuren ist seit längerer Zeit schon deren Umsetzung mit Lipasen bekannt. In der US5518903 ist dieses Prinzip auf N-geschützte β-Aminosäureester mit allerdings wechselndem Erfolg übertragen worden. Während einzig der entsprechende Benzylester von racemischer N-Butoxycarbonyl-β-aminobuttersäure hoch enantioselektiv mittels einer Lipase gespalten werden konnte, lieferten die restlichen eingesetzten Methylester bzw. n-Butylester lediglich ee-Werte im Bereich von max. 70%ee. Es ist dabei festzustellen, dass offensichtlich ein Übergang von einem entsprechenden Methylester zu einem n-Butylester mit einer Verschlechterung des ee-Wertes der hergestellten Säure einhergeht. So ergibt die Esterhydrolyse ausgehend vom n-Butylester der N-Boc-β-aminobuttersäure mit dem Enzym Lipase von der Firma Asahi nach 8 Tagen in 37% Ausbeute einen ee-Wert der entsprechenden Säure von 45%ee. Mit der Lipase PS von Amano erhält man bei der gleichen Reaktion immerhin innerhalb von 7 Tagen mit einer Ausbeute von 41% eine zu 61%ee angereicherte Verbindung. Im Vergleich dazu liefert der entsprechende Methylester 70%ee.

Den kürzlich von Faulconbridge et al. veröffentlichten Ergebnissen ist zu entnehmen, dass die Esterhydrolyse von aromatischen β-Aminosäureethylestern bei pH 8 mit der Lipase PS von Amano in annehmbaren Ausbeuten und sehr guten Enantiomerenüberschüssen erfolgt (Tetrahedron Letters 2000, 41, 2679-81). Das Produkt wird mit einer Enantiomerenreinheit von bis zu 99%ee erhalten, allerdings ist die Synthese, die ausschließlich in wässriger Suspension durchgeführt wurde, mit einigen Nachteilen verbunden. Zum einen hat sich gezeigt, dass zwar die Kristallisation unter diesen Bedingungen selektiv ist, allerdings die Reaktion an sich, wie im Vergleichsbeispiel 2 dokumentiert, zu niedrigeren ee-Werten von 85.1% ee führt. Insgesamt bedeutet dies einerseits einen Ausbeuteverlust aufgrund der Bildung des ungewünschten Enantiomers, zum anderen bedingt es auch die Problematik, dass der ee-Wert in Abhängigkeit von leichten Prozessschwankungen im technischen Maßstab aufgrund veränderter Kristallisationsbedingungen leicht auch unter 99% ee bzw. sogar unter 98% ee fallen kann. Ein möglichst hoher ee-Wert von >98% ee, insb. >99% ee, ist aber für Pharmaanwendungen eine Voraussetzung. Darüber hinaus wäre neben der Durchführung in Suspension auch die Durchführung in rein flüssigem Medium wünschenswert , um z.B. eine gute Enzymabtrennung *via* Ultrafiltration gewährleisten zu können. Optimalerweise sollte in diesem Schritt ebenfalls ein hoher ee-Wert anfallen, was mit dem bisherigen Literaturverfahren nicht realisiert werden kann (siehe dazu auch Vergleichsbeispiele 1 und 2).

Über eine enzymatische Hydrolyse in Gegenwart von einphasigen Reaktionsmedien unter Einsatz organischer Solventien wurde von Nagata et al. berichtet (S. Katayama, N. Ae, R. Nagata, Tetrahedron: Asymmetry 1998, 9, 4295-4299). Dabei wurde ein cyclischer β-Aminosäureester eingesetzt. Die besten Ergebnisse mit Enantioselektivitäten von 94% ee und Umsätzen von 50% bei einer Reaktionszeit von 20h wurden bei Verwendung eines Solvensgemisches, bestehend aus Aceton (90%) und Wasser (10%), erzielt. Mit niedrigeren Wasseranteilen wurden schlechtere Ergebnisse erzielt. Generell hat sich die Verwendung von gut-wasserlöslichen im Vergleich mit schlecht-wassermischbaren Solventien als überlegen herausgestellt. So ergibt die Verwendung von Diisopropylether als organisches Medium, das mit Wasser und 20%-igen Aceton gesättigt wurde, lediglich einen ee-Wert von 58% ee. Im Gegensatz dazu zeigt sich neben Aceton mit THF ein weiteres gut-wasserlösliches Solvens als geeignet (95% ee), wobei hier allerdings lange Reaktionszeiten benötigt werden (96h), um einen einigermaßen vollständigen Umsatz zu erreichen.

Bislang blieb diese erfolgreiche Synthese mit dem Merkmal der Anwesenheit organischer Solventien allerdings auf die Herstellung von cyclischen β-Aminosäureestern begrenzt. Unter den in der Literatur für cyclische β-Aminosäureester angegebenen optimalen Bedingungen (siehe oben) werden bei der Herstellung von den gewünschten Zielverbindungen der offenkettigen Pendants drastisch niedrigere Ausbeuten und nicht akzeptable, lange Reaktionszeiten erhalten (Vergleichsbeispiel 1).

Aufgabe der vorliegenden Erfindung war daher die Angabe eines weiteren Verfahrens zur enzymatischen Herstellung von β-Aminosäuren. Insbesondere sollte dieses Verfahren unter ökonomischen wie ökologischen Gesichtspunkten vorteilhaft in einem technischen Maßstab einsetzbar sein, d.h. in Bezug auf die Umweltverträglichkeit, den Arbeitsschutz und die Robustheit des Prozesses sowie die Raum/Zeit-Ausbeute und Selektivität besonders hervorstechen.

Diese und weitere nicht näher genannte sich jedoch aus dem Stand der Technik in naheliegenderweise ergebende Aufgaben werden gelöst durch ein Verfahren mit den Merkmalen des gegenständlichen Anspruchs 1. Abhängige Ansprüche 2 bis 8 beziehen sich auf bevorzugte Ausführungsformen der vorliegenden Erfindung.

Dadurch, dass man ein Verfahren zur Herstellung enantiomerenangereicherter N-ungeschützter, insbesondere offenkettiger β-Aminosäuren durch enzymatische Hydrolyse eines Enantiomerengemisches von N-ungeschützten, insbesondere offenkettiger β-Aminosäureestern mit einer Hydrolase in einem Zweiphasensystem aus Wasser und einem mit Wasser unter den gegebenen Reaktionsbedingungen zwei Phasen bildenden organischen Lösungsmittel durchführt, gelangt man sehr überraschend, dafür aber in nicht minder vorteilhafter Art und Weise zur Lösung der gestellten Aufgabe. Überraschenderweise werden dabei nicht nur im Vergleich zu den bisher bekannten organisch-wässrigen Systemen erheblich höhere Reaktivitäten, sondern auch gute Enantioselektivitäten erreicht. Die Reaktion im Zweiphasensystem kann sogar so optimiert werden, dass das Produkt mit ≥99% ee anfällt (Beispiel 4). Interessant ist zudem, dass der ee-Wert gemäß erfindungsgemäßem Beispiel 3 (89%ee) im Vergleich zu dem Versuch in rein-wässrigem System deutlich besser ausfällt (Vergleichsbeispiel 2, 81,5%ee). Neben den prozeßtechnischen Vorteilen organischer Solventien besitzt somit dieses Verfahren gegenüber dem wässrigen Standardmedium zudem den Vorteil der Generierung höherer Enantioselektivitäten in den Produkten.

Vorzugsweise werden Verbindungen der allgemeinen Formel (I) in dem gegenständlichen Verfahren eingesetzt, 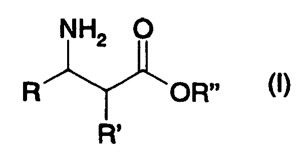 worin
R, R" unabhängig voneinander bedeuten (C₁-C₈)-Alkyl, (C₂-C₈)-Alkenyl, (C₂-C₈)-Alkinyl, (C₃-C₈)-Cycloalkyl, (C₆-C₁₈)-Aryl, (C₇-C₁₉)-Aralkyl, (C₃-C₁₈)-Heteroaryl, (C₄-C₁₉)-Heteroaralkyl, ((C₁-C₈)-Alkyl)₁₋₃-(C₃-C₈)-Cycloalkyl, ((C₁-C₈)-Alkyl)₁₋₃-(C₆-C₁₈)-Aryl, ((C₁-C₈)-Alkyl)₁₋₃-(C₃-C₁₈)-Heteroaryl,
R' bedeutet H, (C₁-C₈)-Alkyl, (C₂-C₈)-Alkenyl, (C₂-C₈)-Alkinyl, (C₃-C₈)-Cycloalkyl, (C₆-C₁₈)-Aryl, (C₇-C₁₉)-Aralkyl, (C₃-C₁₈)-Heteroaryl, (C₄-C₁₉)-Heteroaralkyl, ((C₁-C₈)-Alkyl)_{1- 3}-(C₃-C₈)-Cycloalkyl, ((C₁-C₈)-Alkyl)₁₋₃-(C₆-C₁₈)-Aryl, ((C₁-C₈)-Alkyl)₁₋₃-(C₃-C₁₈)-Heteroaryl.
Im Prinzip ist der Fachmann frei in der Wahl der entsprechenden Estergruppe. Er wird sich bei seiner Auswahl an ökonomischen und reaktionstechnischen Gesichtspunkten orientieren. Günstige Alkohole zur Bildung des Esters sind insbesondere solche, die leicht ggf. durch Destillation aus dem Reaktionsgemisch entfernt werden können. Ganz besonders bevorzugt ist der Einsatz von β-Aminosäurealkyl- oder β-Aminosäurearylestern im erfindungsgemäßen Verfahren. Äußerst bevorzugt ist die Verwendung von entsprechenden n-Propyl-, i-Propyl, n-Butyl, sec-Butyl-, i-Butyl oder tert.-Butylestern.

Die Wahl der Reaktionsparameter ist dem Fachmann ebenfalls freigestellt. Er wird sie anhand von Routineexperimenten für den Einzelfall gesondert ermitteln. Auf jeden Fall eignet sich für das gegenständliche enzymatische Verfahren ein pH-Wertebereich zwischen 4 und 10, vorzugsweise zwischen 6 und 9 und mehr bevorzugt zwischen 7 und 8.5. Um pH 8 hat sich die Lipase PS der Firma Amano als besonders geeignet herausgestellt.

In Hinblick auf die Temperatur liegen prinzipiell die gleichen Vorraussetzungen vor, wie für den pH-Wert. Auch hier kann eine möglichst optimale Temperatur für den Einzelfall ermittelt werden, je nachdem welches Enzym bei welcher Temperatur am optimalsten arbeitet. Für Enzyme aus thermophilen Organismen sind hohe Temperaturen bis 100°C mögliche. Andere wiederum arbeiten optimal erst bei <0°C bis -15°C, ggf. in einer Eismatrix. Bevorzugt sollte die eingestellte Temperatur während der Reaktion im Bereich zwischen 15 und 40°C und mehr bevorzugt zwischen 20 und 30°C liegen.

Die Wahl des einzusetzenden Enzyms obliegt dem Fachmann. Aus Enzyme Catalysis in Organic Synthesis, Ed.: K. Drauz, H. Waldmann, VCH, 1995, S. 165 und der dort zitierten Literatur sind viele geeignete Enzyme auswählbar. Vorzugsweise wird für die Esterhydrolyse eine Lipase genommen, mehr vorzugsweise wird die Lipase PS von Amano aus *Pseudomonas cepacia* eingesetzt.
Für die Anwendung kann das betrachtete Polypeptid in freier Form als homogen aufgereinigte Verbindung oder als rekombinant hergestelltes Enzym verwendet werden. Weiterhin kann das Polypeptid auch als Bestandteil eines intakten Gastorganismus eingesetzt werden oder in Verbindung mit der aufgeschlossenen und beliebig hoch aufgereinigten Zellmasse des Wirtsorganismus.
Möglich ist ebenfalls die Verwendung der Enzyme in immobilisierter Form (Sharma B. P.; Bailey L. F. und Messing R. A. (1982), Immobilisierte Biomaterialien-Techniken und Anwendungen, Angew. Chem. 94, 836-852). Vorteilhafterweise erfolgt die Immobilisierung durch Lyophilisation (Paradkar, V. M.; Dordick, J. S. (1994), Aqueous-Like Activity of α-Chymotrypsin Dissolved in Nearly Anhydrous Organic Solvents, J. Am. Chem. Soc. 116, 5009-5010; Mori, T.; Okahata, Y. (1997), A variety of lipicoated glycoside hydrolases as effective glycosyl transfer catalysts in homogeneous organic solvents, Tetrahedron Lett. 38, 1971-1974; Otamiri, M.; Adlercreutz, P.; Matthiasson, B. (1992), Complex formation between chymotrypsin and ethyl cellulose as a means to solubilize the enzyme in active form in toluene, Biocatalysis 6, 291-305). Ganz besonders bevorzugt ist die Lyophilisation in Gegenwart von oberflächenaktiven Substanzen, wie Aerosol OT oder Polyvinylpyrrolidon oder Polyethylenglycol (PEG) oder Brij 52 (Diethylenglycol-mono-cetylether) (Kamiya, N.; Okazaki, S.-Y.; Goto, M. (1997), Surfactant-horseradish peroxidase complex catalytically active in anhydrous benzene, Biotechnol. Tech. 11, 375-378).
Äußerst bevorzugt ist die Immobilisierung an Eupergit® , insbesondere Eupergit C® und Eupergit 250L® (Röhm) (als Übersicht, siehe: E. Katchalski-Katzir, D. M. Kraemer, J. Mol. Catal. B: Enzym. 2000, 10, 157). Gleichfalls bevorzugt ist die Immobilisierung an Ni-NTA in Kombination mit dem durch Anhängen eines His-Tags (Hexa-Histidin) veränderten Polypeptid (Petty, K.J. (1996), Metal-chelate affinity chromatography In: Ausubel, F.M. et al. eds. Current Protocols in Molecular Biology, Vol. 2, New York: John Wiley and Sons).
Die Verwendung als CLECs ist ebenfalls denkbar (St. Clair, N.; Wang, Y.-F.; Margolin, A. L. (2000), Cofactor-bound cross-linked enzyme crystals (CLEC) of alcohol dehydrogenase, Angew. Chem. Int. Ed. 39, 380-383).
Durch diese Maßnahmen kann es gelingen, aus Polypeptiden, welche durch organische Solventien instabil werden, solche zu generieren, die in Gemischen von wässrigen und organischen Lösungsmitteln bzw. ganz in Organik arbeiten können.

Die gegenständliche Reaktion kann in jedwedem dafür vorgesehenem Reaktionsgefäß durchgeführt werden. Dies sind im einzelnen normale Batchreaktoren, Schlaufenreaktoren, oder ein Enzym-Membran-Reaktor (Bommarius, A. S.; Drauz, K.; Groeger, U.; Wandrey, C.; Membrane Bioreactors for the Production of Enantiomerically Pure α-Amino Acids, in: Chirality in Industry (Hrsg.: Collins, A. N.; Sheldrake, G. N.; Crosby, J.) 1992, John Wiley & Sons, S. 371-397).

Als organische Phase, welche mit Wasser unter den gegebenen Reaktionsbedingungen zwei Phasen bildet somit also nicht oder schlecht wasserlöslich ist und in dem erfindungsgemäßen Verfahren eingesetzt werden kann, kommen sämtliche Arten an organischen, nicht oder schlecht wasserlöslichen Solventien sowie Mischungen daraus in Frage. Insbesondere sind dies Ether, Ketone, Ester, gesättigte oder ungesättigte, lineare oder verzweigtkettige Kohlenwasserstoffe.
Als besonders geeignet haben sich dabei Methyl-tert-butylether (MTBE), Diisopropylether, Essigsäureethylester, Hexan, Heptan, Cyclohexan, Methylcyclohexan und Toluol, sowie entsprechende beliebige Mischungen daraus, erwiesen.

Im Falle des Einsatzes von Enzymen, die ggf. auf wasserunlöslichen Trägermaterialien bzw. Begleitstoffen oder Stabilisatoren adsorbiert vorliegen, hat es sich als vorteilhaft erwiesen, den unlöslichen Träger bzw. Begleitstoff oder Stabilisator vor Einsatz des Enzyms in der Reaktion abzutrennen, damit eine Kontamination des auszufällenden Produktes mit dem unlöslichen Trägermaterial des eingesetzten Enzyms unterbleibt, sofern die Trennung von Enzym und Träger einfach möglich ist. Beispielsweise ist die vorteilhaft anzuwendende Lipase PS der Firma Amano auf Silicaträgern adsorbiert. Hier sollte daher vor der Zugabe der Reaktanden zum Reaktionsmedium eine Filtration der wässrigen Enzymlösung erfolgen, um die Kieselsäuren aus dem Reaktionssystem zu entfernen. Die Aktivität oder Prozessstabilität des Enzyms beeinflusst diese Vorgehensweise in der Regel nicht negativ.

Unter "N-ungeschützt" wird im Rahmen der Erfindung die Tatsache verstanden, dass das β-Stickstoffatom der Säure nicht durch eine unter den Reaktionsbedingungen stabile N-Schutzgruppe blockiert ist. Als solche sind insbesondere die gängigen Schutzgruppen wie Z-, Boc-, Fmoc-, Eoc-, Moc-, Acetyl- etc. zu sehen.

Als (C₁-C₈)-Alkyl sind anzusehen Methyl, Ethyl, n-Propyl, Isopropyl, n-Butyl, Isobutyl, sec-Butyl, tert-Butyl, Pentyl, Hexyl, Heptyl oder Octyl samt aller Bindungsisomeren. Diese können einfach oder mehrfach mit (C₁-C₈)-Alkoxy, (C₁-C₈)-Haloalkyl, OH, Halogen, NH₂, NO₂, SH, S-(C₁-C₈)-Alkyl substituiert sein.

Als (C₂-C₈)-Alkenyl ist mit Ausnahme von Methyl ein wie oben dargestellter (C₁-C₈)-Alkyl-Rest zu verstehen, der mindestens eine Doppelbindung aufweist.

Unter (C₂-C₈)-Alkinyl ist mit Ausnahme von Methyl ein wie oben dargestellter (C₁-C₈)-Alkyl-Rest zu verstehen, der mindestens eine Dreifachbindung aufweist.

Unter (C₁-C₈)-Acyl versteht man einen über eine -C=O-Funktion ans Molekül gebundenen (C₁-C₈)-Alkyl-Rest.

Unter (C₃-C₈)-Cycloalkyl versteht man Cyclopropyl, Cyclobutyl, Cyclopentyl, Cyclohexyl bzw. Cycloheptylreste etc. Diese können mit einem oder mehreren Halogenen und/oder N-, O-, P-, S-atomhaltige Reste substituiert sein und/oder N-, O-, P-, S-atomhaltige Reste im Ring aufweisen, wie z. B. 1-, 2-, 3-, 4-Piperidyl, 1-, 2-, 3-Pyrrolidinyl, 2-, 3-Tetrahydrofuryl, 2-, 3-, 4-Morpholinyl. Dieser kann einfach oder mehrfach mit (C₁-C₈)-Alkoxy, (C₁-C₈)-Haloalkyl, OH, Halogen, NH₂, NO₂, SH, S-(C₁-C₈)-Alkyl, (C₁-C₈)-Acyl, (C₁-C₈)-Alkyl substituiert sein.

Unter einem (C₆-C₁₈)-Arylrest wird ein aromatischer Rest mit 6 bis 18 C-Atomen verstanden. Insbesondere zählen hierzu Verbindungen wie Phenyl-, Naphthyl-, Anthryl-, Phenanthryl-, Biphenylreste. Dieser kann einfach oder mehrfach mit (C₁-C₈)-Alkoxy, (C₁-C₈)-Haloalkyl, OH, Halogen, NH₂, NO₂, SH, S-(C₁-C₈)-Alkyl, (C₁-C₈)-Acyl, (C₁-C₈)-Alkyl substituiert sein.

Ein (C₇-C₁₉)-Aralkylrest ist ein über einen (C₁-C₈)-Alkylrest an das Molekül gebundener (C₆-C₁₈)-Arylrest.

(C₁-C₈)-Alkoxy ist ein über ein Sauerstoffatom an das betrachtete Molekül gebundener (C₁-C₈)-Alkyl-Rest.

(C₁-C₈)-Alkoxycarbonyl ist ein über eine -OC(O)-Funktion an das betrachtete Molekül gebundener (C₁-C₈)-Alkyl-Rest. Dies gilt Synonym für die anderen Oxycarbonylreste.

(C₁-C₈)-Haloalkyl ist ein mit einem oder mehreren Halogenatomen substituierter (C₁-C₈)-Alkyl-Rest.

Ein (C₃-C₁₈)-Heteroarylrest bezeichnet im Rahmen der Erfindung ein fünf-, sechs- oder siebengliedriges aromatisches Ringsystem aus 3 bis 18 C-Atomen, welches Heteroatome wie z. B. Stickstoff, Sauerstoff oder Schwefel im Ring aufweist. Als solche Heteroaromaten werden insbesondere Rest angesehen, wie 1-, 2-, 3-Furyl, wie 1-, 2-, 3-Pyrrolyl, 1-,2-,3-Thienyl, 2-, 3-, 4-Pyridyl, 2-, 3-, 4-, 5-, 6-, 7-Indolyl, 3-, 4-, 5-Pyrazolyl, 2-,4-, 5-Imidazolyl, Acridinyl, Chinolinyl, Phenanthridinyl, 2-, 4-, 5-, 6-Pyrimidinyl. Dieser kann einfach oder mehrfach mit (C₁-C₈)-Alkoxy, (C₁-C₈)-Haloalkyl, OH, Halogen, NH₂, NO₂, SH, S-(C₁-C₈)-Alkyl, (C₁-C₈)-Acyl, (C₁-C₈)-Alkyl substituiert sein.

Unter einem (C₄-C₁₉)-Heteroaralkyl wird ein dem (C₇-C₁₉)-Aralkylrest entsprechendes heteroaromatisches System verstanden.

Als Halogene kommen Fluor, Chlor, Brom und Iod in Frage.

Unter dem Begriff enantiomerenangereichert wird im Rahmen der Erfindung der Anteil eines Enantiomers im Gemisch mit seiner optischen Antipode in einem Bereich von >50 % und <100 % verstanden.

Die dargestellten Strukturen beziehen sich auf alle möglichen Diastereomere und Enantiomere und deren Gemische, die möglich sind.

Die genannten Literaturstellen gelten als von der Offenbarung dieser Erfindung mitumfasst.

### Experimentelle Beispiele:

### Vergleichsbeispiel 1:

Es werden 9.2 mmol der racemischen Verbindung *rac*-3-Amino-3-phenylpropionsäureethylester (1.79 g) in 50 mL eines Lösungsmittelgemisches, bestehend aus 25 mL Wasser und 25 mL Aceton als organische Solvenskomponente, aufgenommen und mittels automatischer pH-Statierung durch Zugabe von 1 M Natronlauge (bezug von Fa. Merck) die Lösung auf einen pH-Wert von pH 8.2 eingestellt. Bei Erreichen einer Reaktionstemperatur von 20 °C werden 200 mg Amano Lipase PS (*Pseudomonas cepacia*; Bezug durch Fa. Amano Enzymes, Inc.) zugegeben, um die Reaktion zu starten. Nach einer Reaktionszeit von 3, 5 und 24 Stunden wird die gebildete Umsatzrate des gebildeten (S)-3-Amino-3-phenylpropionsäure bestimmt. Dabei wird ein Umsatz von 1.8% nach 3 Stunden, 2.0% nach 5 Stunden und 5.5% nach 24 Stunden ermittelt. Der Wert der Enantioselektivität wurde aufgrund des unbefriedigenden Verlaufs der Reaktion angesicht des niedrigen Umsatzes nicht bestimmt. Die Umsatzbestimmung erfolgte *via* HPLC.

### Vergleichsbeispiel 2:

Es werden 9.2 mmol der racemischen Verbindung *rac*-3-Amino-3-phenylpropionsäureethylester (1.79 g) in 50 mL Wasser aufgenommen und mittels automatischer pH-Statierung durch Zugabe von 1 M Natronlauge (Bezug durch Fa. Merck) die Lösung auf einen pH-Wert von pH 8.2 eingestellt. Um den Ester restlos in Lösung zu bringen, werden noch 3 mL Aceton zum Lösen hinzugefügt. Bei Erreichen einer Reaktionstemperatur von 20 °C werden 200 mg Amano Lipase PS (*Pseudomonas cepacia*; Bezug durch Fa. Amano Enzymes, Inc.) zugegeben, um die Reaktion zu starten. Nach einer Reaktionszeit von 3 und 6 Stunden wird die gebildete Umsatzrate sowie nach 6 Stunden die Enantioselektivität des gebildeten (*S*)-3-Amino-3-phenylpropionsäure bestimmt. Dabei wird ein Umsatz von 18.5% nach 3 Stunden bzw. 37.8% nach 6 Stunden und eine Enantioselektivität von 85.1% ee (nach 6 Stunden) ermittelt. Die Umsatz- und Enantioselektivitätsbestimmung erfolgte *via* HPLC.

### Beispiel 3:

Es werden 9.2 mmol der racemischen Verbindung *rac*-3-Amino-3-phenylpropionsäureethylester (1.79 g) in 50 mL eines zweiphasigen Lösungsmittelgemisches, bestehend aus 25 mL Wasser und 25 mL Methyl-tert-butylether (MTBE) als organische Solvenskomponente, aufgenommen und mittels automatischer pH-Statierung durch Zugabe von 1 M Natronlauge (Bezug durch Fa. Merck) das Zweiphasensystem auf einen pH-Wert von pH 8.2 eingestellt. Bei Erreichen einer Reaktionstemperatur von 20 °C werden 200 mg Amano Lipase PS (*Pseudomonas cepacia*; Bezug durch Fa. Amano Enzymes, Inc.) zugegeben um die Reaktion zu starten. Nach einer Reaktionszeit von 3, 5 und 24 Stunden wird die gebildete Umsatzrate des gebildeten (S)-3-Amino-3-phenylpropionsäure bestimmt. Dabei wird ein Umsatz von 23.5% nach 3 Stunden bzw. ein quantitativer Umsatz von ca. ≥50% nach 15 Stunden und eine Enantioselektivität von 89.0% ee (nach 15 Stunden) ermittelt. Die Umsatz- und Enantioselektivitätsbestimmung erfolgte *via* HPLC.

### Beispiel 4:

Es werden 81 mL Wasser vorgelegt und dazu 1.45 g Amano Lipase PS (*Pseudomonas cepacia*; Bezug durch Fa. Amano Enzymes, Inc.) zugegeben. Anschließend filtriert man den ungelösten Feststoff ab. Die als Filtrat resultierende wäßrige Enzymlösung wird mit 81 mL Methyl-tert-butylether (MTBE) als organische Solvenskomponente versetzt. Das entstandene Zweiphasensystem wird mittels automatischer pH-Statierung durch Zugabe von 1 M Natronlauge (Bezug durch Fa. Merck) auf pH 8.2 eingestellt. Bei Erreichen einer Temperatur von 20 °C werden dann 188.2 mmol der racemischen Verbindung *rac*-3-Amino-3-phenylpropionsäure-*n*-propylester (39.0 g) hinzugegeben, und die Reaktion gestartet. Die Reaktionszeit beträgt 15 Stunden, wobei ein weißer Niederschlag, bestehend aus dem gewünschten Produkt (S)-3-Amino-3-phenylpropionsäure, anfällt. Nach einer Reaktionszeit von 15 Stunden werden 160 mL Aceton zur Komplettierung der Fällung zugefügt, ca. 45 Minuten nachgerührt und der Feststoff abfiltriert. Der Feststoff wird mehrmals mit wenig Aceton gewaschen und anschließend im Vakuum getrocknet. Es werden 12.91 g der gewünschten (*S*)-3-Amino-3-phenylpropionsäure erhalten, entsprechend einer Ausbeute von 41.6%. Die Enantioselektivität für das Produkt liegt bei 99.6% ee. Die Enantioselektivitätsbestimmung erfolgte *via* HPLC. Für die chemische Reinheit wurde 98.8% ermittelt (ermittelt *via* Titration). Die Struktur des Produkts wurde zusätzlich *via* NMR-Spektroskopie bestätigt.

## Patentansprüche

1. Verfahren zur Herstellung enantiomerenangereicherter N-ungeschützter, insbesondere offenkettiger β-Aminosäuren durch enzymatische Hydrolyse eines Enantiomerengemisches von N-ungeschützten, insbesondere offenkettigen β-Aminosäureestern mit einer Hydrolase, wobei die Hydrolyse in einem Zweiphasensystem aus Wasser und einem mit Wasser unter den gegebenen Reaktionsbedingungen zwei Phasen bildenden organischen Lösungsmittel geschieht.

2. Verfahren nach Anspruch 1,
**dadurch gekennzeichnet, dass**
ein β-Aminosäurealkylester oder β-Aminosäurearylester eingesetzt wird.

3. Verfahren nach Anspruch 2,
**dadurch gekennzeichnet, dass**
ein ensprechender n-Propyl-, i-Propyl, n-Butyl, sec-Butyl-, i-Butyl oder tert.-Butylester eingesetzt wird.

4. Verfahren nach einem oder mehreren der vorhergehenden Ansprüche,
**dadurch gekennzeichnet, dass**
der pH-Wert der Reaktion zwischen 4 und 10, vorzugsweise zwischen 6 und 9 und mehr bevorzugt zwischen 7 und 8,5 liegt.

5. Verfahren nach einem oder mehreren der vorhergehenden Ansprüche,
**dadurch gekennzeichnet, dass**
die Temperatur bei der Reaktion zwischen -15 und +100°C, vorzugsweise zwischen +15 und +40°C und mehr bevorzugt zwischen +20 und +30°C liegt.

6. Verfahren nach einem oder mehreren der vorhergehenden Ansprüche,
**dadurch gekennzeichnet, dass**
man eine Lipase, vorzugsweise die Lipase PS aus *Pseudomonas cepacia* einsetzt.

7. Verfahren nach einem oder mehreren der vorhergehenden Ansprüche,
**dadurch gekennzeichnet, dass**
man die Reaktion in einem Enzym-Membran-Reaktor durchführt.

8. Verfahren nach einem oder mehreren der vorhergehenden Ansprüche,
**dadurch gekennzeichnet, dass**
man als organisches Lösungsmittel Ether, Ketone, Ester, gesättigte oder ungesättigte lineare oder verzweigtkettige Kohlenwasserstoffe einsetzt.

## Claims

1. Process for preparing enantiomer-enriched N-unprotected, in particular open-chain β-amino acids by enzymatic hydrolysis of an enantiomeric mixture of N-unprotected, in particular open-chain β-amino acid esters with a hydrolase, where the hydrolysis takes place in a two-phase system composed of water and of an organic solvent which forms two phases with water under the given reaction conditions.

2. Process according to Claim 1, **characterized in that** a β-amino acid alkyl ester or β-amino acid aryl ester is employed.

3. Process according to Claim 2, **characterized in that** a corresponding n-propyl, i-propyl, n-butyl, sec-butyl, i-butyl or tert-butyl ester is employed.

4. Process according to one or more of the preceding claims, **characterized in that** the pH of the reaction is between 4 and 10, preferably between 6 and 9 and more preferably between 7 and 8.5.

5. Process according to one or more of the preceding claims, **characterized in that** the temperature in the reaction is between -15 and +100°C, preferably between +15 and +40°C and more preferably between +20 and +30°C.

6. Process according to one or more of the preceding claims, **characterized in that** a lipase, preferably lipase PS from *Pseudomonas cepacia,* is employed.

7. Process according to one or more of the preceding claims, **characterized in that** the reaction is carried out in an enzyme membrane reactor.

8. Process according to one or more of the preceding claims, **characterized in that** ethers, ketones, esters, saturated or unsaturated linear or branched-chain hydrocarbons are employed as organic solvent.

## Revendications

1. Procédé pour la préparation de β-aminoacides enrichis en énantiomères, non protégés sur l'atome d'azote, en particulier à chaîne ouverte, par hydrolyse enzymatique à l'aide d'une hydrolase d'un mélange d'énantiomères d'esters de β-aminoacides non protégés sur l'atome d'azote, en particulier à chaîne ouverte, l'hydrolyse étant effectuée dans un système en deux phases constitué d'eau et d'un solvant organique formant deux phases avec l'eau dans les conditions réactionnelles données.

2. Procédé selon la revendication 1, **caractérisé en ce qu'**on utilise des esters alkyliques de β-aminoacides ou des esters aryliques de β-aminoacides.

3. Procédé selon la revendication 2, **caractérisé en ce qu'**on utilise un ester n-propylique, isopropylique, n-butylique, sec-butylique, isobutylique ou tert-butylique correspondant.

4. Procédé selon une ou plusieurs des revendications précédentes, **caractérisé en ce que** le pH de la réaction est compris entre 4 et 10, de préférence entre 6 et 9, et de façon particulièrement préférée entre 7 et 8,5.

5. Procédé selon une ou plusieurs des revendications précédentes, **caractérisé en ce que** la température dans la réaction est comprise entre -15 et +100 °C, de préférence entre +15 et +40 °C, et de façon particulièrement préférée entre +20 et +30 °C.

6. Procédé selon une ou plusieurs des revendications précédentes, **caractérisé en ce qu'**on utilise une lipase, de préférence la lipase de *Pseudomonas cepacia.*

7. Procédé selon une ou plusieurs des revendications précédentes, **caractérisé en ce qu'**on effectue la réaction dans un réacteur à membrane-enzyme.

8. Procédé selon une ou plusieurs des revendications précédentes, **caractérisé en ce qu'**on utilise comme solvant organique des éthers, des cétones, des esters, des hydrocarbures saturés ou insaturés, linéaires ou à chaîne ramifiée.
